# EUROPÄISCHE PATENTSCHRIFT

(11) **EP 0 937 704 B1**
(45) Veröffentlichungstag und Bekanntmachung des Hinweises auf die Patenterteilung: **30.10.2002**
(21) Anmeldenummer: 99100965.5
(22) Anmeldetag: 20.01.1999
(51) Int. Cl.: C07C 231/02

(54) **Verfahren zur Herstellung von Acylglutamat-Lösungen**
Process for the preparation of solutions of acylglutamate
Procédé pour la préparation de solutions d'acylglutamate

(30) Priorität: 17.02.1998 DE 19806512
(43) Veröffentlichungstag der Anmeldung: 25.08.1999
(73) Patentinhaber: Clariant GmbH, 65929 Frankfurt am Main (DE)
(72) Erfinder: Hingott, Thomas Dr., 65830 Kriftel (DE); Hess, Joachim, 65719 Hofheim (DE)

(56) Entgegenhaltungen:
- US-A- 2 463 779
- DATABASE WPI Section Ch, Week 197643 Derwent Publications Ltd., London, GB; Class B05, AN 1976-80620X XP002122446 & SU 325 837 A (DISINFECT STERILISA), 30. März 1976 (1976-03-30)
- LEYDET A ET AL: "Polyanion inhibitors of human immunodeficiency virus. Part IV. - Polymerized anionic surfactants: influence of the density and distribution of anionic groups on the antiviral activity" BIOORGANIC & MEDICINAL CHEMISTRY LETTERS,GB,OXFORD, Bd. 6, Nr. 4, Seite 397-402 XP004135044 ISSN: 0960-894X

## Beschreibung

Acylglutamate zählen zu den milden Tensiden und zeichnen sich durch eine sehr gute biologische Abbaubarkeit, geringe Aquatoxizität und eine milde, gute bis ausgezeichnete Haut- und Schleimhautverträglichkeit aus. Breite Verwendung finden Acylglutamate als pflegende Komponente, insbesondere das Cocoylglutamat-Natriumsalz in kosmetischen Reinigungspräparaten.

Die Synthese von Acylglutamaten beruht auf der bekannten Schotten-Baumann Reaktion, also der Umsetzung von Fettsäurechloriden mit der Aminogruppe des Glutaminsäure-Natriumsalzes.

Um das lipophile Fettsäurechlorid mit der hydrophilen Aminosäure bzw. dem zugrunde liegenden Salz in wäßrigem Medium zur Reaktion zu bringen, ist nach den bisherigen Verfahrensweisen die Zugabe eines organischen Lösungsmittels wie beispielsweise Aceton, Methylethylketon, Dioxan, Tetrahydrofuran, t.-Butanol oder Cyclohexan notwendig (US-3 758 525). Nachteilig bei diesem Verfahren ist die Tatsache, daß das organische Lösungsmittel in sehr zeitaufwendigen und kostenintensiven Verfahren aus dem Reaktionsgemisch entfernt werden muß.

Die Aufgabe der vorliegenden Erfindung besteht darin, ein Verfahren zur Herstellung von Acylglutamaten zu finden, das ohne Lösungsmittel arbeitet.

Überraschenderweise wurde gefunden, daß bei Zugabe von Tensiden zur wäßrigen Reaktionsmischung auf ein organisches Lösungsmittel verzichtet werden kann. Durch die Gegenwart eines Tensids kann die Konzentration des Reaktionsproduktes Acylglutamat in der fertigen wäßrigen Lösung auf 25 bis 40, insbesondere 25 bis 35 Gew.-% gesteigert werden.

Gegenstand der Erfindung ist somit ein Verfahren zur Herstellung von Acylglutamaten der allgemeinen Formel 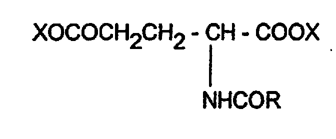 wobei R C₆-C₃₆-, vorzugsweise C₁₀-C₁₈-Alkyl oder C₆-C₃₆-, vorzugsweise C₁₀-C₁₈-Alkenyl und X ein Alkali- oder Ammonium-Ion bedeuten, durch Umsetzung eines Glutaminsäuresalzes der Formel 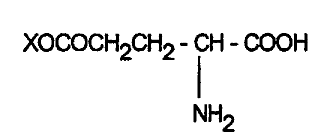 mit Alkalihydroxid und anschließende Umsetzung mit einem Säurechlorid der Formel

RCOCl

in Gegenwart von Wasser und einem anionischen oder zwitterionischen Tensid, wobei das anionische oder zwitterionische Tensid vor dem Säurechlorid zugegeben wird.

Das Verfahren wird vorzugsweise so durchgeführt, daß man zunächst eine ca. 10 bis 30 %ige wäßrige Glutamat-Lösung herstellt und das Tensid sowie Alkalihydroxid zugibt. Die Menge an Tensid ist so bemessen, daß die fertige Acylglutamat-Lösung im allgemeinen 2 bis 50, vorzugsweise 3 bis 30, insbesondere 5 bis 25 Gew.-% Tensid enthält. Die Menge an Alkali beträgt 1,0 bis 1,03 mol Alkali pro Mol Glutaminsäure-Salz. Diese Lösung wird auf ca. 10 bis 20°C abgekühlt und dann gibt man langsam die äquimolare Menge an Fettsäurechlorid zu. Gleichzeitig gibt man noch soviel Alkalihydroxid zu, um einen pH-Wert der Reaktionsmischung von ca. 12,2 bis 12,6 einzuhalten. Die Temperatur soll dabei vorzugsweise 10 bis 20°C nicht überschreiten. Nach Zugabe des Fettsäurechlorids rührt man noch ca. 2 Stunden ohne Kühlung nach und stellt dann den pH-Wert des Reaktionsgemisches auf 9 bis 10.

Als Tenside eignen sich alle anionischen und zwitterionischen Tenside, die nicht mit dem Säurechlorid reagieren und die gegen Alkali beständig sind.

Als anionische Tenside können ganz allgemein Alkyl-Sulfate, -Sulfonate, -Carboxylate, -Phosphate, und deren Mischungen eingesetzt werden. Die anionischen Tenside, die für die vorliegende Erfindung geeignet sind, sind wasserlöslich oder in Wasser dispergierbar. Diese anionischen Tenside enthalten als Kation Natrium, Kalium, Calcium, Magnesium, Ammonium oder substituierte Ammonium-kationen, einschließlich Mono-, Di- oder Triethanolammoniumkationen.

Speziell in Frage kommen Alkylsulfate der Formel ROSO₃M, wobei R C₁₀-C₂₄-, vorzugsweise C₁₀-C₂₀-, insbesondere C₁₂-C₁₈-Alkyl und M Wasserstoff oder ein Kation ist, z.B. ein Alkalimetallkation (z.B. Natrium, Kalium, Lithium) oder Ammonium oder substituiertes Ammonium, z.B. Methyl-, Dimethyl- und Trimethylammoniumkationen und quaternäre Ammoniumkationen, wie Tetramethylammonium- und Dimethylpiperidiniumkationen.

Für das erfindungsgemäße Verfahren besonders geeignete Tenside sind wasserlösliche Alkylethersulfate der Formel RO(A)ₘSO₃M, worin R C₁₀-C₂₄-, bevorzugt C₁₂-C₂₀-, besonders bevorzugt C₁₂-C₁₈-Alkyl darstellt. A ist eine Ethoxyoder Propoxyeinheit, m ist größer als 0, typischer Weise eine Zahl zwischen ca. 0,5 und ca. 6, besonders bevorzugt zwischen ca. 0,5 und ca. 3 und M ist Wasserstoff oder ein Kation, wie z.B. ein Metallkation (z.B. Natrium, Kalium, Lithium, Calcium, Magnesium, etc.), Ammonium oder ein substituiertes Ammoniumkation. Spezifische Beispiele für substituierte Ammoniumkationen sind Methyl-, Dimethyl-, Trimethylammonium- und quaternäre Ammoniumkationen, wie Tetramethylammonium und Dimethylpiperidiniumkationen. Als Beispiele seien genannt C₁₂-C₁₈-Alkyl-polyethoxysulfate mit jeweils 1; 2,25; 3 oder 4 Mol Ethylenoxid und Natrium oder Kalium als Kation.

Ein weiteres geeignetes anionisches Tensid, das erfindungsgemäß eingesetzt werden kann, ist Alkylbenzolsulfonat. Die Alkylgruppe kann entweder gesättigt oder ungesättigt, verzweigt oder linear sein. Die bevorzugten Alkylbenzolsulfonate enthalten lineare Alkylketten mit 9 bis 25 Kohlenstoffatomen, bevorzugt von 10 bis 13 Kohlenstoffatome, das Kation ist Natrium, Kalium, Ammonium, Mono-, Di- oder Triethanolammonium, Calcium oder Magnesium und Mischungen davon.

Ebenso können sekundäre Alkansulfonate eingesetzt werden, wobei die Alkylgruppe entweder gesättigt oder ungesättigt, verzweigt oder linear sein kann. Die Sulfogruppe ist statistisch über die gesamte C-Kette verteilt, wobei die primären Methylgruppen am Kettenanfang und Kettenende keine Sulfonatgruppe besitzen. Die bevorzugten sekundären Alkansulfonate enthalten lineare Alkylketten mit 9 bis 25 Kohlenstoffatomen, bevorzugt mit 10 bis 20 Kohlenstoffatomen und besonders bevorzugt mit 13 bis 17 Kohlenstoffatomen. Das Kation ist Natrium, Kalium, Ammonium, Mono-, Di- oder Triethanolammonium, Calcium oder Magnesium und Mischungen davon. Natrium als Kation ist der Einfachheit halber bevorzugt.

Weitere einsetzbare Tenside sind Carboxylate, z.B. Fettsäureseifen. Die Seifen können gesättigt oder ungesättigt sein und können verschiedene Substituenten, beispielsweise Alpha-Sulfonatgruppen enthalten. Bevorzugt enthalten sie lineare gesättigte oder ungesättigte Kohlenwasserstoffreste als hydrophobe Komponente. Üblicherweise enthalten die hydrophoben Komponenten 6 bis 30 Kohlenstoffatome, bevorzugt 10 bis 18 Kohlenstoffatome. Das Kation ist ein Alkalikation, z.B. Natrium oder Kalium, ein Erdalkalikation, z.B. Calcium oder Magnesium oder Ammonium oder substituiertes Ammonium einschließlich Mono-, Di- und Triethanolammonium.

Weitere anionische Tenside sind Salze von Acylaminocarbonsäuren, die durch Umsetzung von Fettsäurechloriden mit Natriumsarkosinat im alkalischen Medium entstehen (Acylsarcosinate) sowie die Salze von Alkylsulfamidocarbonsäuren und die Salze von Alkyl- und Alkylarylethercarbonsäuren. Ebenso können die Acylglutamate selbst als Tenside bei dem erfindungsgemäßen Verfahren eingesetzt werden.
Weiter einsetzbare anionische Tenside sind C₈-C₂₄-Olefinsulfonate, sulfonierte Polycarboxylsäuren, hergestellt durch Sulfonierung der Pyrrolyseprodukte von Erdalkalimetallcitraten, wie z.B. beschrieben in GB-A-1 082 179, Alkylglycerinsulfate, Fettacylglycerinsulfate, Oleylglycerinsulfate, Alkylphenolethersulfate, primäre Paraffinsulfonate, Alkylphosphate, Alkyletherphosphate, Isethionate, wie Acylisethionate, N-Acyltauride, Alkylsuccinamate, Sulfosuccinate, Monoester der Sulfosuccinate (besonders gesättigte und ungesättigte C₁₂-C₁₈-Monoester) und Diester der Sulfosuccinate (besonders gesättigte und ungesättigte C₁₂-C₁₈-Diester), Sulfate von Alkylpolysacchariden und Alkylpolyethoxycarboxylate wie die der Formel RO(CH₂CH₂)ₖCH₂COO-M⁺ worin R ein C₈-C₂₂-Alkyl, k eine Zahl von 0 bis 10 und M ein lösliches Salz bildendes Kation ist. Harzsäuren oder hydrierte Harzsäuren, wie Rosin oder Tallölharze und Tallölharzsäuren sind ebenfalls einsetzbar.

Zwitterionische Tenside, die bei dem erfindungsgemäßen Verfahren eingesetzt werden können, sind ganz allgemein aliphatische sekundäre oder tertiäre Amine mit einem linearen oder verzweigten C₈-C₁₈-Alkylrest und die eine anionische, wasserlösliche Gruppe, wie z.B. eine Carboxy-, Sulfonat-, Sulfat-, Phosphat- oder Phosphonatgruppe enthalten.

Typische Beispiele für zwitterionische Tenside sind Alkylbetaine, Alkylamidbetaine, Aminopropionate, Aminoglycinate oder amphotere Verbindungen der Formel

R¹CONR⁴(CH₂)ₙN^{⊖}R²R³CH₂Z X^{⊖}

worin R¹ C₈-C₂₂-Alkyl- oder -Alkenyl, R² Wasserstoff oder CH₂CO₂M, R³ CH₂CH₂OH oder CH₂CH₂OCH₂COOM, R⁴ Wasserstoff, CH₂CH₂OH oder CH₂CH₂COOM, Z CO₂M oder CH₂CO₂M, n 2 oder 3, bevorzugt 2, M Wasserstoff oder ein Kation wie Alkalimetall, Erdalkalimetall, Ammoniak oder Alkanolammonium bedeutet.

Bevorzugte zwitterionische Tenside dieser Formel sind Monocarboxylate und Dicarboxylate. Beispiele hierfür sind Cocoamphocarboxypropionat, Cocoamidocarboxypropionsäure, Cocoamphocarboxyglycinat (auch als Cocoamphodiacetat bezeichnet) und Cocoamphoacetat.

Weitere bevorzugte zwitterionische Tenside sind Alkyldimethylbetaine und Alkyldipolyethoxybetaine mit einem Alkylrest, der linear oder verzweigt sein kann, mit ca. 8 bis ca. 22 Kohlenstoffatomen, bevorzugt mit 8 bis 18 Kohlenstoffatomen und besonders bevorzugt mit ca.12 bis ca. 18 Kohlenstoffatomen. Diese Verbindungen werden z.B. von der Clariant AG unter dem Handelsnamen ®Genagen LAB vermarktet.

Von allen zuvor genannten Tensiden sind die Acylglutamate, insbesondere Cocoylglutamat, Alkylethersulfat, insbesondere Laurylethersulfat und Alkylamidopropylbetaine, insbesondere Cocoamidopropylbetain bevorzugt. Weiterhin ist es bevorzugt, mit einer Mischung verschiedener Tenside zu arbeiten.

### Beispiel 1: Herstellung von C₁₂-Acylglutamat, Di-Natriumsalz

In einem mit N₂ inertisierten Reaktor werden 440 g Wasser, 196,7 g Mononatriumglutamat, 128,5 g 33 %ige Natronlauge und 102,5 g ®Genapol LRO (flüssig), vorgelegt und auf 10 bis 20°C abgekühlt.
innerhalb von 8 Stunden werden mit Hilfe einer Dosierpumpe 245,9 g Laurylfettsäurechlorid und gleichzeitig 120 g bis 130 g 33 %ige Natronlauge zugetropft. Während der Reaktionszeit muß ein pH-Wert von 12,2 bis 12,6 eingehalten werden. Gleichzeitig muß die Temperatur der Reaktionsmischung während der Zutropfphase zwischen 10 und 20°C liegen. In der Nachrührzeit (ca. 2 h) wird durch Zugabe von NaOH ein pH-Wert zwischen 11,0 und 12,0 eingestellt. Abkühlen auf 10 bis 20°C ist in der Nachrührzeit nicht mehr erforderlich. Am Ende der Nachrührzeit wird das Reaktionsprodukt auf 60°C erwärmt und mit Salzsäure ein pH-Wert von 9 bis 10 eingestellt.

### Beispiel 2: Herstellung von C₁₂-C₁₈-Cocoylglutamat, Di-Natriumsalz

In einem mit N₂ inertisierten Reaktor werden 440 g Wasser, 196,7 g Mononatriumglutamat, 128,5 g 33 %ige Natronlauge und 102,5 g Genapol LRO (flüssig) vorgelegt und auf 10 bis 20°C abgekühlt.
Innerhalb von 8 Stunden werden mit Hilfe einer Dosierpumpe 252,3 g Cocosfettsäurechlorid und gleichzeitig 120 g bis 130 g 33 %ige Natronlauge zugetropft. Während der Reaktionszeit muß ein pH-Wert von 12,2 bis 12,6 eingehalten werden. Gleichzeitig muß die Temperatur der Reaktionsmischung während der Zutropfphase zwischen 10 und 20°C liegen. In der Nachrührzeit (ca. 2 h) wird durch Zugabe von NaOH ein pH-Wert zwischen 11,0 und 12,0 eingestellt. Am Ende der Nachrührzeit wird das Reaktionsprodukt auf 60°C erwärmt und mit Salzsäure ein pH-Wert von 9 bis 10 eingestellt.

### Beispiel 3: Herstellung von C₁₂-C₁₈-Cocoylglutamat, Di-Natriumsalz

In einem mit N₂ inertisierten Reaktor werden 440 g Wasser, 196,7 g Mononatriumglutamat, 128,5 g 33 %ige Natronlauge und 120,4 g ®Hostapon KCG vorgelegt und auf 10 bis 20°C abgekühlt.
Innerhalb von 8 Stunden werden mit Hilfe einer Dosierpumpe 252,3 g C₁₂-C₁₈-Cocosfettsäurechlorid und gleichzeitig 120 g bis 130 g 33 %ige Natronlauge zugetropft. Während der Reaktionszeit muß ein pH-Wert von 12,2 bis 12,6 eingehalten werden. Gleichzeitig muß die Temperatur der Reaktionsmischung während der Zutropfphase zwischen 10 und 20°C liegen. In der Nachrührzeit (ca. 2 h) wird durch Zugabe von NaOH ein pH-Wert zwischen 11,0 und 12,0 eingestellt. Am Ende der Nachrührzeit wird das Reaktionsprodukt auf 60°C erwärmt und mit Salzsäure ein pH-Wert von 9 bis 10 eingestellt.

### Beispiel 4: Herstellung von C₁₀-Acylglutamat, Di-Natriumsalz

In einem mit N₂ inertisierten Reaktor werden 440 g Wasser, 196,7 g Mononatriumglutamat, 128,5 g 33 %ige Natronlauge und 115,5 g ®Genagen CAB vorgelegt und auf 10 bis 20°C abgekühlt.
Innerhalb von 8 Stunden werden mit Hilfe einer Dosierpumpe 216,3 g Decansäurechlorid und gleichzeitig 120 g bis 130 g 33 %ige Natronlauge zugetropft. Während der Reaktionszeit muß ein pH-Wert von 12,2 bis 12,6 eingehalten werden. Gleichzeitig muß die Temperatur der Reaktionsmischung während der Zutropfphase zwischen 10 und 20°C liegen. In der Nachrührzeit (ca. 2 h) wird durch Zugabe von NaOH ein pH-Wert zwischen 11,0 und 12,0 eingestellt. Am Ende der Nachrührzeit wird das Reaktionsprodukt auf 60°C erwärmt und mit Salzsäure ein pH-Wert von 9 bis 10 eingestellt.

### Beispiel 5: Herstellung von C₁₂-C₁₄-Cocoylglutamat, Di-Natriumsalz

In einem mit N₂ inertisierten Reaktor werden 440 g Wasser, 196,7 g Mononatriumglutamat, 128,5 g 33 %ige Natronlauge und 102,5 g Genapol LRO, flüssig vorgelegt und auf 10 bis 20°C abgekühlt.
Innerhalb von 8 Stunden werden mit Hilfe einer Dosierpumpe 249,5 g C₁₂-C₁₄-Cocosfettsäurechlorid und gleichzeitig 120 g bis 130 g 33 %ige Natronlauge zugetropft. Während der Reaktionszeit muß ein pH-Wert von 12,2 bis 12,6 eingehalten werden. Gleichzeitig muß die Temperatur der Reaktionsmischung während der Zutropfphase zwischen 10 und 20°C liegen. In der Nachrührzeit (ca. 2 h) wird durch Zugabe von NaOH ein pH-Wert zwischen 11,0 und 12,0 eingestellt. Am Ende der Nachrührzeit wird das Reaktionsprodukt auf 60°C erwärmt und mit Salzsäure ein pH-Wert von 9 bis 10 eingestellt.

| Chemische Bezeichnung der Handelsnamen | |
|---|---|
| Genapol LRO, flüssig | 27,0 % C₁₂-C₁₄-Alkyldiglycol-ether-sulfat, Natriumsalz in Wasser, Clariant GmbH |
| Hostapon KCG | 25 % Acylglutamat Mono-natriumsalz in Wasser, Clariant GmbH |
| Genagen CAB | 30 % Cocoamidopropylbetain, Clariant GmbH |

## Patentansprüche

1. Verfahren zur Herstellung von Acylglutamaten der allgemeinen Formel 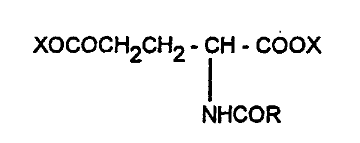 wobei R C₆-C₃₆-Alkyl oder C₆-C₃₆-Alkenyl und X ein Alkali- oder Ammonium-Ion bedeuten, durch Umsetzung eines Glutaminsäuresalzes der Formel 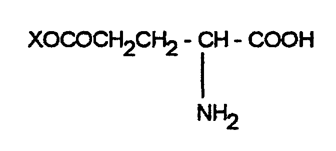 mit Alkalihydroxid und anschließende Umsetzung mit einem Säurechlorid der Formel
RCOCl
in Gegenwart von Wasser und einem anionischen oder zwitterionischen Tensid, **dadurch gekennzeichnet, daß** das anionische oder zwitterionische Tensid vor dem Säurechlorid zugegeben wird.

2. Verfahren nach Anspruch 1, **dadurch gekennzeichnet, daß** man die Reaktion in Gegenwart von 2 bis 50, vorzugsweise 3 bis 30, insbesondere 5 bis 25 Gew.-% Tensid durchführt

3. Verfahren nach Anspruch 1, **dadurch gekennzeichnet, daß** man die Reaktion in Gegenwart eines Acylglutamats, Alkylethersulfats undloder Alkylamidopropylbetains durchführt.

## Claims

1. A process for the preparation of acylglutamates of the formula 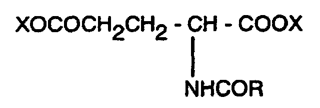 in which R is C₆-C₃₆-alkyl or C₆-C₃₆-alkenyl and X is an alkali metal ion or ammonium ion, by reaction of a glutamic acid salt of the formula 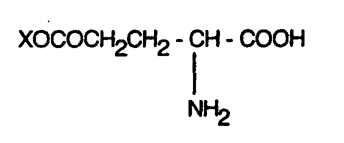 with alkali metal hydroxide and subsequent reaction with an acid chloride of the formula
RCOCl
in the presence of water and an anionic or zwitterionic surfactant, which comprises adding the anionic or zwitterionic surfactant before the acid chloride.

2. The process as claimed in claim 1, wherein the reaction is carried out in the presence of from 2 to 50% by weight, preferably from 3 to 30% by weight, in particular from 5 to 25% by weight, of surfactant.

3. The process as claimed in claim 1, wherein the reaction is carried out in the presence of an acylglutamate, alkyl ether sulfate and/or alkylamidopropylbetaine.

## Revendications

1. Procédé pour la préparation de glutamates d'acyle de formule générale 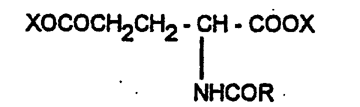 dans laquelle R représente un groupe alkyle en C₆-C₃₆ ou alcényle en C₆-C₃₆ et X représente un ion alcalin ou ammonium, par réaction d'un sel d'acide glutamique de formule 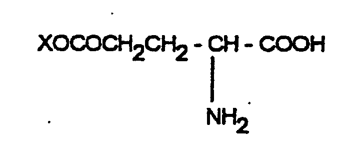 avec un hydroxyde de métal alcalin et ensuite réaction avec un chlorurè d'acide de formule
RCOCl
en présence d'eau et d'un agent tensioactif anionique ou zwitterionique, **caractérisé en ce que** l'agent tensioactif anionique ou zwitterionique est ajouté avant le chlorure d'acide.

2. Procédé selon la revendication 1, **caractérisé en ce qu'**on réalise la réaction en présence de 2 à 50, de préférence de 3 à 30, en particulier de 5 à 25 % en poids d'agent tensioactif.

3. Procédé selon la revendication 1, **caractérisé en ce qu'**on réalise la réaction en présence d'un glutamate d'acyle, d'un éthersulfate d'alkyle et/ou d'alkylamidopropylbétalne.
